(19) Europäisches Patentamt
European Patent Office
Office européen des brevets

(11) EP 1 999 775 B1

(12) **EUROPÄISCHE PATENTSCHRIFT**

(45) Veröffentlichungstag und Bekanntmachung des Hinweises auf die Patenterteilung:
**29.06.2011 Patentblatt 2011/26**

(21) Anmeldenummer: **07723422.7**

(22) Anmeldetag: **20.03.2007**

(51) Int Cl.:
***H01J 33/00*** *(2006.01)* ***A61L 2/00*** *(2006.01)*

(86) Internationale Anmeldenummer:
**PCT/EP2007/002458**

(87) Internationale Veröffentlichungsnummer:
**WO 2007/107331 (27.09.2007 Gazette 2007/39)**

(54) **VORRICHTUNG UND VERFAHREN ZUR EIGENSCHAFTSÄNDERUNG DREIDIMENSIONALER FORMTEILE MITTELS ELEKTRONEN SOWIE ANWENDUNG DES VERFAHRENS**

DEVICE AND METHOD FOR ALTERING THE CHARACTERISTICS OF THREE-DIMENSIONAL SHAPED PARTS USING ELECTRONS AND USE OF SAID METHOD

DISPOSITIF ET PROCÉDÉ DE MODIFICATION DES PROPRIÉTÉS DE CORPS FORMÉS TRIDIMENSIONNELS AU MOYEN D’ÉLECTRONS ET APPLICATION DU PROCÉDÉ

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **20.03.2006 DE 102006012668**

(43) Veröffentlichungstag der Anmeldung:
**10.12.2008 Patentblatt 2008/50**

(73) Patentinhaber: **Fraunhofer-Gesellschaft zur Förderung der angewandten Forschung e.V.
80686 München (DE)**

(72) Erfinder:
- **BARTEL, Rainer
01324 Dresden (DE)**
- **KIRCHHOFF, Volker
01829 Wehlen (DE)**
- **MATTAUSCH, Gösta
01454 Ullersdorf (DE)**
- **RÖDER, Olaf
01326 Dresden (DE)**
- **KUBUSCH, Jörg
01279 Dresden (DE)**

(56) Entgegenhaltungen:
**WO-A-94/28573 WO-A-99/39750
WO-A-99/39751**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 999 775 B1

**Beschreibung**

Die Erfindung betrifft eine Vorrichtung und ein Verfahren zum Modifizieren von Stoffeigenschaften an der Oberfläche und im Randbereich dreidimensionaler Formteile mittels der Energie von Elektronen. Des Weiteren werden Anwendungen des Verfahrens aufgezeigt.

Mittels Elektronen kann Energie räumlich und zeitlich determiniert in Materialien eingetragen werden, um deren Stoffeigenschaften an der Oberfläche, in der Randschicht oder im Volumen zu verändern. Die dazu benötigten Elektronen werden in Elektronenbeschleunigern erzeugt, formiert und beschleunigt, ehe sie über ein meist ebenes Elektronenaustrittsfenster aus dem Hochvakuum an ein höheres Druckniveau im Prozessraum geführt werden. Dabei ist meist eine konstante Elektronendichte über der gesamten Ausdehnung des Elektronenaustrittsfensters gewünscht. Nach dem Durchdringen der Gasschicht (zum Beispiel Luft) in der Distanz zwischen Elektronenaustrittsfenster und Produkt erreichen die Elektronen die zu behandelnde Produktoberfläche.

Als Elektronenbeschleuniger werden Flächenstrahlerzeuger, auch Bandstrahler genannt, oder Axialstrahlerzeuger eingesetzt. Ein als Axialstrahlerzeuger ausgebildeter Elektronenbeschleuniger nach dem Stand der Technik umfasst zusätzlich einen Elektronenstrahlablenkraum mit einem Strahlablenksystem, mittels dem ein erzeugter Elektronenstrahl periodisch über das gesamte Elektronenaustrittsfenster und im zeitlichen Mittel in allen Teilbereichen des Fensters mit annähernd gleicher Verweildauer abgelenkt wird.

Dreidimensionale Formteile, wie beispielsweise Verpackungen, medizinische Implantate, OP-Bestecke, Prothesen aus verschiedenen Materialien (zum Beispiel Kunststoff, Papier, Metall, Keramik) werden in unterschiedlichen Branchen (zum Beispiel Verpackungsindustrie, Pharmazie, Medizintechnik, Kunststoffindustrie) eingesetzt. Für bestimmte Anwendungen ist eine Eigenschaftsänderung (zum Beispiel Sterilisation, Oberflächenfunktionalisierung, Vernetzung, Härtung) der gesamten Oberfläche und der Randschicht des Formteiles erforderlich.

Stand der Technik

Es ist bekannt, Eigenschaften der gesamten Oberfläche von dreidimensionalen Formteilen mittels Elektronenenergie zu beeinflussen, indem ein Formteil in mehreren Durchläufen (DE 199 42 142 A1) und in veränderter Position an einem Elektronenaustrittsfenster vorbeigeführt wird. Bekannte Vorrichtungen zum Erzeugen von Elektronen für das Modifizieren von Formteileigenschaften sind derart gestaltet, dass über das gesamte Elektronenaustrittsfenster eine annähernd gleiche Elektronenenergiedichte erzeugt und abgegeben wird.

Durch das Verändern der Position eines Formteils wird sichergestellt, dass die gesamte Formteiloberfläche mit Elektronenenergie beaufschlagt wird. Ein Nachteil derartiger Vorrichtungen besteht darin, dass ein Mehrfachdurchlauf mit einem relativ hohen Zeitaufwand verbunden ist. Das Verändern der Position des Formteils zwischen den einzelnen Durchläufen kann auch nicht wahllos erfolgen, sondern muss derart abgestimmt sein, dass einzelne Oberflächenbereiche in der Summe nicht mit unterschiedlichen Elektronenenergiedichten beaufschlagt werden, was zu unterschiedlichen Eigenschaften führen würde.

Nach dem Stand der Technik wird die gesamte Oberfläche eines dreidimensionalen Formteils während nur eines Durchlaufs mittels Elektronenenergie modifiziert, indem mehrere (mindestens zwei oder drei) Elektronenaustrittsfenster derart angeordnet werden, dass diese den Querschnitt des Formteils umschließen, wobei das Formteil zwischen diesen Elektronenaustrittsfenstern hindurchgeführt und somit die gesamte dreidimensionale Oberfläche mit Elektronen beaufschlagt wird.

Von der Firma LINAC Technologies (Technische Beschreibung "ELECTRON BEAM SURFACE STERILISATION SYSTEM 200 KeV - The Ke VAC S") ist eine Vorrichtung zum Sterilisieren der Oberfläche von Formteilen mittels Elektronenenergie bekannt, bei der drei Elektronenbeschleuniger derart angeordnet sind, dass deren zugehörige Elektronenaustrittsfenster ein Volumen mit dem Querschnitt eines gleichschenkligen Dreiecks umschließen, durch das die zu sterilisierenden Formteile in einem Durchlauf hindurchgeführt werden. Mit derartigen Vorrichtungen wird zwar gegenüber bekannten Lösungen, bei denen ein Formteil in mehreren Durchläufen mit Elektronen beaufschlagt wird, der zeitliche Aufwand verringert, der technische Aufwand ist jedoch wegen des Einsatzes von drei Elektronenbeschleunigern sehr groß.

Es sind ähnliche Anordnungen von drei Elektronenaustrittsfenstern bekannt, bei denen die Elektronen jedoch nur mittels eines Elektronenbeschleunigers erzeugt und mit Hilfe eines Ablenksystems auf die drei Elektronenaustrittsfenster aufgeteilt werden.

Alle bekannten Lösungen mit drei Elektronenaustrittsfenstern nutzen den Vorteil, dass sich die Elektronenbeschleuniger durch deren Dreiecksanordnung nicht oder vernachlässigbar gegenseitig beeinflussen, dass heißt, dass die beschleunigten Elektronen eines Elektronenbeschleunigers keine erheblichen Energieanteile auf die jeweils anderen Elektronenbeschleuniger abgeben. Dies ist erforderlich, um den im Elektronenaustrittsfenster absorbierten Energieanteil und damit dessen Betriebstemperatur auf ein unterkritisches Maß zu begrenzen. Bei Überschreitung der Materialeinsatztemperatur würde das empfindliche Material der Fensterabdeckung anderenfalls durch die mechanische Belastung des von außen anliegenden Atmosphärendruckes, im Verhältnis zum Hochvakuum im Inneren des Strahlerzeugers, zerstört. Für die üblicherweise in Elektronenaustrittsfenstern eingesetzte Titanfolie darf eine Maximaltemperatur von etwa 400 °C keinesfalls überschritten werden. Für den Dauerbetrieb geht man von maximal 200-250 °C aus.

Die Anordnung von nur zwei gegenüberliegenden Elektronenaustrittsfenstern ist ebenfalls bekannt. Bei der technologisch erforderlichen geringen Distanz zwischen den Elektronenaustrittsfenstern wird dabei ein erheblicher Energieanteil in das gegenüberliegende Elektronenaustrittsfenster eingebracht, was je nach Konstruktion eine Temperaturerhöhung um den Faktor 2 bis 5 zur Folge hat. Die notwendige Begrenzung der Maximaltemperatur kann nur durch proportionale Begrenzung des Strahlstromes erreicht werden. Dies jedoch begrenzt die Leistungsfähigkeit des Gesamtsystems.

Eine weitere Möglichkeit zur Begrenzung der Temperatur zweier gegenüberliegender Elektronenaustrittsfenster ist die Anordnung eines zusätzlichen Absorbers wie z. B. eines (zumindest teiltransparenten) Transportbandes zwischen den Elektronenaustrittsfenstern (US 2,741,704). Ein erheblicher Energieanteil fällt dann auf den Absorber, was die Einstrahlung zusätzlicher Energie auf das gegenüberliegende Elektronenaustrittsfenster begrenzt.

Ebenfalls bekannt ist die Lösung, zwei Elektronenaustrittsfenster gegenüberliegend und seitlich in Produkt-Transportrichtung versetzt anzuordnen. Dadurch wird die Einstrahlung von Leistung in den jeweils gegenüberliegenden Elektronenbeschleuniger und damit dessen Überhitzung verhindert.

Bei den bekannten Einrichtungen, bei denen zwei und mehr Elektronenaustrittsfenster ein Formteil umschließen und bei denen über ein gesamtes Elektronenaustrittsfenster eine annähernd gleiche Elektronenenergiedichte abgegeben und ein Formteil nur in einem Durchlauf mit Elektronen beaufschlagt wird, können einzelne Oberflächenteilbereiche des Formteils in Abhängigkeit von dessen Geometrie und dem daraus resultierenden unterschiedlichen Abstand der Oberflächenteilbereiche von einem Elektronenaustrittsfenster mit einer unterschiedlichen Dosis (Energie pro Flächeneinheit oder Energie pro Masseeinheit) an Elektronenenergie beaufschlagt werden.

Um eine bestimmte Eigenschaft an einem Formteil zu realisieren, ist eine bestimmte Dosis an Elektronenenergie erforderlich. Zweckmäßigerweise wird die Leistung der Elektronenerzeuger derart eingestellt, dass an jenen Oberflächenbereichen, an denen die geringste Dosis ankommt, die dort ankommende Dosis genau oder mindestens der Dosis entspricht, die für das Modifizieren der Eigenschaft erforderlich ist. Alle anderen Oberflächenbereiche des Formteils werden zwangsläufig mit einer erhöhten Dosis beaufschlagt. Diese erhöhte Dosis an Energie wird auch als Überdosis bezeichnet. Je höher die Überdosis in einzelnen Bereichen eines Formteils ist, umso stärker weichen die Eigenschaften in diesen Bereichen von den Zielparametern ab. Eine Überdosis an Elektronenenergie wirkt sich jedoch nicht nur negativ auf die zu modifizierenden Eigenschaften eines Formteils aus, sondern kann auch zu unerwünschten oder gar prozessschädlichen Nebenwirkungen durch das Bilden unerwünschter Reaktionsprodukte (zum Beispiel Ozon) im Prozessgas (zum Beispiel Luft) führen.

Ein als Überdosisfaktor bezeichneter Parameter gibt an, um das Wievielfache eine erforderliche Dosis zum Einstellen einer gewünschten Eigenschaft überschritten wird. Mit den bekannten Einrichtungen werden in Abhängigkeit von der Geometrie zu modifizierender Formteile in einzelnen Oberflächenbereichen Überdosisfaktoren erreicht, die für viele Anwendungen nicht akzeptabel sind, um hinreichend gleichmäßige Eigenschaften über die gesamte Oberfläche zu realisieren und was auch die bereits genannten unerwünschten Nebenwirkungen mit sich bringt.

Für die Erzielung hoher Produktivitäten ist eine angepasst hohe Transportgeschwindigkeit der Formteile erforderlich. Wegen der Proportionalität von Transportgeschwindigkeit und Strahlstrom erfordert die Erzielung einer technologisch vorgegebenen Mindestdosis (für den Aufgabenbereich der Sterilisation liegt diese z. B. bei 25 kGy) eine mit der Geschwindigkeit proportionale Erhöhung des Strahlstromes, was zur überproportionalen Erhöhung der Betriebstemperatur der Elektronenaustrittsfenster führt. Für den Fall der gegenüberliegenden Anordnung zweier Elektronenbeschleuniger ohne zusätzlichen Absorber oder seitlichen Versatz der Systeme existiert derzeit keine praxistaugliche Lösung.

## Aufgabenstellung

Der Erfindung liegt daher das technische Problem zugrunde, eine Vorrichtung und ein Verfahren zu schaffen, mittels denen die Nachteile des Standes der Technik überwunden werden. Insbesondere sollen Vorrichtung und Verfahren geeignet sein, Eigenschaften dreidimensionaler Formteile mit einem geringen zeitlichen und technischen Aufwand derart zu modifizieren, dass eine hinreichend gleichmäßige Modifizierung der gesamten Oberfläche oder einer Randschicht der Formteile erfolgt und dennoch keine die Produktivität begrenzenden Nachteile aus der Gesamtanordnung der Elektronenbeschleuniger aufweisen. Dabei soll der Überdosisfaktor so gering sein, dass dieser den technisch/technologischen Anforderungen der Formteile entspricht.

Die Lösung des technischen Problems ergibt sich durch die Gegenstände mit den Merkmalen der Patentansprüche 1 und 19. Weitere vorteilhafte Ausgestaltungen der Erfindung ergeben sich aus den abhängigen Patentansprüchen.

Nach dem Stand der Technik ging man bisher davon aus, dass mindestens zwei Elektronenaustrittsfenster mit seitlichem Versatz oder mit dazwischen liegendem Absorber oder mit begrenztem Strahlstrom bzw. drei Elektronenaustrittsfenster erforderlich sind, um den Querschnittsumfang eines dreidimensionalen Formteils in einem Durchlauf vollständig mit Elektronen beaufschlagen und gewünschte Eigenschaftsveränderungen herbeiführen zu können. Erfindungsgemäß wird gezeigt, dass eine aus einer gegenüberliegenden Anordnung von Elektronenaustrittsfenstern resultierende Begrenzung des Strahlstromes nicht erforderlich ist und zusätzlich eine Beaufschlagung einer Formteiloberfläche mit einer annähernd gleichmäßig verteilten Energiedosis möglich ist.

Eine erfindungsgemäße Vorrichtung zur Eigenschaftsänderung eines dreidimensionalen Formteils mittels Elektronen umfasst mindestens einen Elektronenbeschleuniger zum Erzeugen von beschleunigten Elektronen und zwei Elektronenaustrittsfenster, wobei die beiden Elektronenaustrittsfenster gegenüberliegend angeordnet sind. Gemeinsam mit mindestens einem Reflektor begrenzen die beiden Elektronenaustrittsfenster einen Prozessraum, in welchem das Formteil mit Elektronen beaufschlagt wird. Die Elektronenaustrittsfenster sind dabei so weit voneinander beabstandet, dass eine Beeinflussung eines Elektronenaustrittsfensters durch die abgestrahlte Energie des gegenüberliegenden Elektronenaustrittsfensters vernachlässigbar ist. Der hierfür erforderliche Abstand weist im Wesentlichen eine Abhängigkeit von der Beschleunigungsspannung der Elektronen, der Dicke und Dichte der Folie eines Elektronenaustrittsfensters und der Dichte des Gases zwischen den Elektronenaustrittsfenstern auf.

Der Nachteil, dass bei einem derartigen Abstand nicht mehr alle Oberflächenbereiche (insbesondere die Bereiche, die weitgehend senkrecht zur Oberfläche der Elektronenaustrittsfenster verlaufen) des zu modifizierenden Formteils hinreichend mit Elektronen beaufschlagt werden, wird ausgeglichen, indem der Reflektor derart geformt und angeordnet ist, dass Elektronen (insbesondere aus den Randbereichen der Elektronenaustrittsfenster), welche das Formteil nicht treffen würden, vom Reflektor auf die Oberflächenbereiche des Formteils reflektiert werden, die ein Defizit bei der Beaufschlagung mit Elektronen aufweisen.

Des Weiteren umfasst eine erfindungsgemäße Vorrichtung ein Sensorsystem, mittels dem die Energiedichteverteilung im Prozessraum über mindestens eine räumliche Dimension erfasst werden kann. In Abhängigkeit von den hierbei erfassten Daten kann dann die über die Elektronenaustrittsfenster abgegebene Energiedichte derart gesteuert werden, dass innerhalb der Dimension, in welcher die Energiedichteverteilung erfasst wurde, eine gleichmäßige Beaufschlagung mit Elektronen auf die Formteiloberfläche erfolgt.

Eine derartige Vorrichtung ist besonders geeignet für Formteile, die einen weitestgehend runden, ovalen oder trapezförmigen Querschnitt aufweisen. Damit können jedoch auch Formteile mit andersartigem Querschnitt modifiziert werden.

Zum Erzeugen der beschleunigten Elektronen kann ein Elektronenbeschleuniger verwendet werden, von dem die Elektronen mit einer entsprechenden Umlenksteuerung auf die beiden Elektronenaustrittsfenster aufgeteilt werden. Alternativ kann jedoch auch jedem Elektronenaustrittsfenster ein separater Elektronenbeschleuniger zugeordnet sein. Als Elektronenbeschleuniger sind sowohl Flächenbeschleuniger, auch als Bandstrahler bezeichnet, als auch Axialstrahlerzeuger geeignet.

Bei einer parallelen Ausrichtung zweier gegenüberliegender, eben ausgebildeter Elektronenaustrittsfenster mit optimalem Abstand und erfindungsgemäßer Anordnung eines Reflektorsystems konnten während der Elektronenbehandlung von Formteilen mit weitestgehend trapezförmigem Querschnitt Überdosisfaktoren realisiert werden, die unter dem Wert 4 lagen, wohingegen bei der Behandlung gleicher Formteile in Vorrichtungen nach dem Stand der Technik mit drei Elektronenaustrittsfenstern oder mit zwei gegenüberliegenden Elektronenaustrittsfenstern und dazwischen liegendem Absorber Überdosisfaktoren von weit über 4 hingenommen werden mussten. Gegenüber bekannten Lösungen werden somit bei hoher Produktivität einerseits Energie gespart und andererseits die Oberfläche des dreidimensionalen Produktes vor strahlenchemischen Schäden bewahrt sowie aufgrund des geringeren Ausstoßes von Ozon prozessschädigende Nebenwirkungen reduziert.

Eine Ausführungsform der Erfindung umfasst zwei Reflektoren, welche den Prozessraum begrenzen und spiegelsymmetrisch gegenüberliegend angeordnet sind. Dabei kann jeder der beiden Reflektoren aus einer Vielzahl von Teilreflektoren bestehen.

Bei einer bevorzugten Ausführungsform sind die Reflektoren gleichzeitig Bestandteil des Sensorsystems zum Erfassen einer Energiedichteverteilung. Hierbei kann beispielsweise eine Anzahl von Reflektoren bzw. Teilreflektoren, die bevorzugt aus einem Material mit hoher Ordnungszahl (beispielsweise Gold, Wolfram oder Molybdän) bestehen, über einen Widerstand elektrisch mit der elektrischen Masse oder einem anderen elektrischen Potential verbunden sein. Elektronen, die nicht von einem Reflektor/Teilreflektor reflektiert werden, bilden dann einen Stromfluss aus, so dass über einem zum Reflektor/Teilreflektor zugehörigen Widerstand eine Spannung erfasst werden kann. Über die Werte der erfassten Spannung an den einzelnen Reflektoren/Teilreflektoren kann dann auch eine entsprechende Aussage bezüglich der Energiedichte der von einem Reflektor reflektierten Elektronen getroffen und entsprechende Regelschritte bezüglich einer gleichmäßigen Energiedichteverteilung eingeleitet werden.

Besonders vorteilhaft ist es, wenn auf diese Weise die Energiedichteverteilungen in x-, y- und z-Richtung eines rechtwinkligen Koordinatensystems erfasst und entsprechend ausgewertet werden.

Mit Hilfe einer derartigen Kombination von Reflektoren und Sensorsystem kann beispielsweise auch erfasst werden, ob sich ein Formteil im Prozessraum befindet. In Abhängigkeit davon kann das Erzeugen von Elektronen gesteuert werden, so dass die Leistung der Elektronenbeschleuniger beispielsweise dann, wenn sich ein Formteil im Prozessraum befindet, auf einen prozessspezifischen Wert eingestellt wird und anderenfalls abgesenkt bzw. auf Null reduziert wird.

Bei einer erfindungsgemäßen Einrichtung kann der maximal auftretende Überdosisfaktor bzw. ein gleichmäßiges Beaufschlagen der Formteiloberfläche mit Elektronen weiter optimiert werden, indem die beiden Elektronenaustrittsfenster in Abhängigkeit von der Geometrie eines zu behandelnden Formteils in einem Winkel zueinander ausgerichtet werden, so dass möglichst viele Oberflächenabschnitte des Formteils mit annähernd gleichem Maß vom jeweils Energie einstrahlenden Elektronenaustrittsfenster beabstandet sind.

Neben eben geformten Elektronenaustrittsfenstern können diese beispielsweise auch zum Formteil hin konkav ausgebildet oder auch der Geometrie des Formteils angepasst sein, was ebenfalls bewirkt, dass möglichst viele Oberflächenabschnitte des Formteils mit annähernd gleichem Maß vom jeweils Energie einstrahlenden Elektronenaustrittsfenster beabstandet sind, wodurch geringere Überdosisfaktoren erzielt werden können.

Bei einer Ausführungsform umfasst ein Elektronenerzeuger eine Einrichtung, mittels der die über die Fläche mindestens eines Elektronenaustrittsfensters abgegebene Elektronenenergiedichte derart steuerbar ist, dass über einzelne Teilbereiche des Elektronenaustrittsfensters unterschiedliche Elektronenenergiedichten abgegeben werden. So kann beispielsweise in den Teilbereichen des Fensters, in denen dem Fenster Oberflächenbereiche eines Formteils mit großem Abstand gegenüberliegen, die Elektronenenergiedichte erhöht werden gegenüber Teilbereichen des Fensters, in denen dem Fenster Oberflächenbereiche des Formteils mit geringem Abstand gegenüberliegen, so dass möglichst alle Oberflächenbereiche des Formteils die gleiche Dosis absorbieren und somit über die gesamte Oberfläche in der zu modifizierenden Bearbeitungstiefe (Oberfläche oder Randschicht) gleichmäßige Eigenschaften ausgebildet werden. Als Mittel zum Modifizieren der Elektronenenergiedichte über einzelne Teilbereiche eines Elektronenaustrittsfensters können innerhalb eines Flächenstrahlerzeugers (ohne elektromagnetische Strahlablenkung) liegende konstruktive, in die Elektronenoptik eingreifende Systeme, wie Blenden, Hilfselektroden oder die Temperatur der Katode beeinflussende Bauteile verwendet werden, welche die Verteilung des Elektronenstromes beeinflussen.

Eine weitere Möglichkeit besteht in der Anordnung von Mitteln außerhalb des Elektronenbeschleunigers, insbesondere von magnetischen oder/und elektrischen Systemen, welche die Richtung der beschleunigten Elektronen beeinflussen.

Eine weitere Ausführungsform der Erfindung zeichnet sich dadurch aus, dass mindestens ein Elektronenaustrittsfenster bewegbar angeordnet ist. So kann dieses Elektronenaustrittsfenster beispielsweise zu Beginn, wenn ein Formteil in den Prozessraum zwischen beiden Fenstern eingebracht wird, zur Stirnseite des Formteils hin verkippt werden, um stirnseitig das Beaufschlagen mit Elektronen zu verbessern. Beim weiteren Transport des Formteils durch den Prozessraum hindurch kann das Fenster dann in Richtung paralleler Ausrichtung zum gegenüberliegenden Fenster und beim Verlassen des Prozessraumes in Richtung Rückseite des Formteiles verkippt werden. Es ist jedoch auch möglich, dass mit dem Fenster andere Bewegungsformen ausgeführt werden. So kann das Fenster beispielsweise zeitweise in Bewegungsrichtung des Formteils mitgeführt werden.

Eine weitere Optimierung bei der Zielstellung, die Eigenschaften gleichmäßig über die gesamte Oberfläche eines Formteils zu modifizieren, ist mittels einer Einrichtung möglich, die über magnetische oder/und elektrische Ablenksysteme nicht nur den Punkt steuert, an dem ein Elektron ein Elektronenaustrittsfenster verlässt, sondern auch die Austrittsrichtung des Elektrons an diesem Punkt. Damit lassen sich noch gezielter bestimmte Oberflächenbereiche des Formteils mit Elektronen beaufschlagen.

Bei einer bevorzugten Ausführungsform ist zumindest ein Elektronenaustrittsfenster als vakuumdichte Folie und somit als Druckbarriere zwischen Strahlführungsraum und Prozessraum ausgebildet. Alternativ kann ein Elektronenaustrittsfenster auch als gasdurchlässige Druckstufenanordnung zwischen Elektronengenerator und Prozessraum ausgebildet sein. Ein erfindungsgemäßes Verfahren zur Eigenschaftsänderung eines dreidimensionalen Formteils mittels Elektronen zeichnet sich dadurch aus, dass mittels mindestens einem Elektronenbeschleuniger Elektronen erzeugt, beschleunigt und über die Fläche von zwei gegenüberliegenden Elektronenaustrittsfenstern abgestrahlt werden, wobei die beiden Elektronenaustrittsfenster und mindestens ein Elektronen-Reflektor einen Prozessraum begrenzen, in welchem die Oberfläche oder eine Randschicht des Formteils mit Elektronen beaufschlagt wird, wobei mittels eines Sensorsystems eine Energiedichteverteilung innerhalb des Prozessraumes zumindest über eine räumliche Dimension erfasst und der Abstand der Elektronenaustrittsfenster derart eingestellt wird, dass eine Beeinflussung eines Elektronenaustrittsfensters durch die abgestrahlte Energie des gegenüberliegenden Elektronenaustrittsfensters vernachlässigbar ist.

Vorteilhaft wird der Abstand der Elektronenaustrittsfenster in Abhängigkeit von der Beschleunigungsspannung der Elektronen und der Dicke und der Dichte der Elektronenaustrittsfenster eingestellt.

Bei einer Ausführungsform wird der Abstand a der Elektronenaustrittsfenster in einem Bereich eingestellt, der sich aus der Formel

$$a = f * \frac{6{,}67 * 10^{-7} \frac{(Ub * k_1)^{5/3}}{\rho_W} * k_2 - \rho_F * d_F}{\rho_G}$$

$\alpha$ = Distanz der Elektronenbeschleuniger
Ub = Beschleunigungsspannung
$\rho_W$ = Dichte von Wasser
$\rho_G$ = Dichte des Mediums zwischen den Elektronenaustrittsfenstern
$\rho_F$ = Dichte der Fensterfolie
$d_F$ = Dicke der Fensterfolie
$k_1 = 1*V^{-1}$
$k_2 = 1*(g/m^2)^{-1}$
f = Abstandsfaktor ($0{,}5 < f < 1{,}5$) ergibt.

Der Bereich für den Abstand a ergibt sich hierbei aus dem Wertebereich des Abstandsfaktors f, wobei aus einem Abstandsfaktor mit einem Wert von 1 ein optimaler Rechenwert für den Abstand a resultiert.

Für das Bestrahlen eines Formteils innerhalb des Prozessraumes zwischen den beiden Elektronenaustrittsfenstern stehen verschiedene alternative Möglichkeiten zur Verfügung.

So kann ein Formteil mit konstanter Geschwindigkeit durch den Prozessraum geführt und währenddessen mit Elektronen beaufschlagt werden.

Alternativ besteht auch die Möglichkeit, dass ein Formteil in den Prozessraum geführt und dort im stationären Zustand durch einen einmaligen oder mehrfachen Bestrahlungsvorgang mit Elektronen beaufschlagt wird.

Bei einer weiteren alternativen Ausführungsform wird ein Formteil im so genannten Step-and-Repeat-Modus mit Elektronen beaufschlagt. Hierunter ist zu verstehen, dass das Formteil derart in den Prozessraum geführt wird, dass zumindest ein Stück des Formteils in den Prozessraum ragt. Im stationären Zustand wird das Formteil dann mit Elektronen aus den Elektronenaustrittsfenstern beaufschlagt. Hieran schließt sich ein erneuter Transportschritt an, in welchem das Formteil eine weitere Strecke in bzw. durch den Prozessraum bewegt wird. Im bewegungslosen Zustand erfolgt dann wiederum ein Bestrahlungsschritt, in welchem das Formteil erneut mit Elektronen beaufschlagt wird. So wechseln sich Transport- und Bestrahlungsschritte ab, bis das Formteil vollständig durch den Prozessbereich bewegt wurde. Ein jeweiliger Transportschritt kann dabei derart ausgeführt werden, dass die einzelnen Oberflächenbereiche, die nach den jeweiligen Transportschritten mit Elektronen beaufschlagt werden, aneinandergrenzen oder sich bei einer alternativen Variante überlappen.

Schließlich ist auch eine Modifikation eines Formteils durchführbar, indem das Formteil im Prozessraum um eine sich zwischen die beiden Elektronenaustrittsfenster erstreckende Achse rotiert und währenddessen durch einen einmaligen oder mehrfachen Bestrahlungsvorgang mit Elektronen beaufschlagt wird.

Erfindungsgemäße Verfahren können beispielsweise zur Sterilisation von Verpackungen und Produkten der Pharmaindustrie und der Medizintechnik, zur Sterilisation/Desinfektion oder Entkeimung von Produkten wie Früchten, Eiern oder anderen Naturprodukten, zur Modifikation von Kunststoffen, Härten von Beschichtungen oder zur Sterilisation/Desinfektion von Gegenständen verwendet werden.

Ausführungsbeispiel

Die Erfindung wird nachfolgend anhand eines bevorzugten Ausführungsbeispiels näher erläutert. Die Fig. zeigen:

Fig. 1 eine schematische Querschnittsdarstellung einer erfindungsgemäßen Vorrichtung;
Fig. 2 eine graphische Darstellung der Tiefendosisverteilung der von den gegenüber- liegenden Elektronenaustrittsfenstern 5a und 5b aus Fig.1 abgestrahlten Elektronen;
Fig. 3 eine schematische Darstellung eines Sensorsystems, umfassend die Reflektoren 7a1 und 7b1 aus Fig. 1.

In Fig. 1 ist schematisch eine Vorrichtung 1 zur Elektronenbehandlung zum Zwecke der Sterilisation der Oberfläche eines Formteils 2 im Querschnitt dargestellt. Formteil 2 ist ein länglicher Gegenstand mit trapezförmigem Querschnitt. Vorrichtung 1 besteht aus zwei als Flächenstrahlerzeuger ausgebildeten Elektronenbeschleunigern 3a, 3b, die jeweils einen Elektronenbeschleunigungsraum 4a, 4b und ein Elektronenaustrittsfenster 5a, 5b umfassen. Hierbei sind die Elektronenaustrittsfenster als 11 μm dicke Titanfolie ausgebildet. Die Elektronenbeschleuniger 3a, 3b sind derart angeordnet, dass die eben geformten Elektronenaustrittsfenster 5a, 5b parallel gegenüberliegend ausgerichtet sind. Zwischen beiden Elektronenaustrittsfenstern 5a, 5b wird Formteil 2 auf einem auf Höhe des Elektronenaustrittsfensters 5b unterbrochenen und in Fig. 1 gepunktet dargestellten Förderbandsystem 6 kontinuierlich hindurchgeführt und dabei dessen gesamte Oberfläche mit Elektronenenergie beaufschlagt. An den schrägen Seitenflächen des Formteils 2 würde dabei jeweils die geringste Energiedosis an den am weitesten von den Elektronenaustrittsfenstern entfernten Punkten übertragen, was durch die Anordnung von Elektronenreflektoren 7a1, 7b1, 7a2, 7b2 aus Gold kompensiert wird. Dies geschieht, indem die ungenutzten Randstrahlen 8a1, 8a2, 8b1, 8b2 des jeweiligen Elektronenstrahles der beiden Elektronenbeschleuniger 3a, 3b auf den jeweils nächstgelegenen Elektronenreflektor treffen, dort reflektiert werden und durch die Winkelanordnung der Reflektoren in den Bereich der niedrigsten Dosis auf das Formteil geführt werden. Aus einer derartigen Gesamtanordnung resultiert eine Energiedosis auf der gesamten Oberfläche oder auch in einer Randschicht des Formteiles mit einem minimalen Überdosisfaktor, einer maximalen Ausnutzung des Elektronenstromes und einem Minimum an in der Luftstrecke entstehendem reaktiven Ozon.

Der in der Anordnung gewählte Abstand der beiden Elektronenaustrittsfenster 5a und 5b entspricht weitgehend folgendem Zusammenhang:

$$a = f * \frac{6{,}67 * 10^{-7} \dfrac{(Ub * k_1)^{5/3}}{\rho_W} * k_2 - \rho_F * d_F}{\rho_G}$$

$\alpha$ = Distanz der Elektronenbeschleuniger
Ub = Beschleunigungsspannung
$\rho_W$ = Dichte von Wasser
$\rho_G$ = Dichte des Mediums zwischen den Elektronenaustrittsfenstern
$\rho_F$ = Dichte der Fensterfolie
$d_F$ = Dicke der Fensterfolie
$k_1 = 1*V^{-1}$
$k_2 = 1 *(g/m^2)^{-1}$
f = Abstandsfaktor (0,5 < f < 1,5), wobei f=1 einen optimalen Abstand definiert.

Bei 11 $\mu$m dicken Titanfolien als Elektronenaustrittsfenster 5a, 5b und dem Medium Luft (hier angenommen 1188 g/m$^3$) zwischen diesen Elektronenaustrittsfenstern ergibt sich ein optimaler Abstand von 196 mm.

Fig. 2 zeigt beispielhaft die Tiefendosisverteilung der erfindungsgemäßen Anordnung zweier Elektronenbeschleuniger gemäß Fig. 1 mit einer Dicke der Elektronenaustrittsfensterfolien (Titan) von 11 $\mu$m bei einer Beschleunigungsspannung von 150 kV und einem optimalen Abstand der Elektronenaustrittsfenster von 196 mm. Kurve 10 stellt die Verteilung der durch Elektronenbeschleuniger 3a erzeugten Energiedosis über der Eindringtiefe der Elektronen dar. Die Energie der Elektronen ist an Punkt 11 bei einer Flächenmasse von 280g/m$^2$ (entspricht bei einer Dichte von 1000 g/m$^3$ einer dem Zahlenwert gemäßen Eindringtiefe in mm - beim angegebenen Fall also 280 mm), auf Null abgefallen. Erst in dieser Distanz befindet sich das Elektronenaustrittsfenster 5b, dessen Flächenmasse als schraffierte Fläche 12 in Figur 2 dargestellt ist. Die gleichen Bedingungen ergeben sich für den Elektronenbeschleuniger 3b, dessen erzeugte Energiedosis als Kurve 13 dargestellt, an Punkt 14 auf Null abgebaut ist (in der Darstellung Fig. 2 bei etwa 50 g/m$^2$). Die Distanz der Punkte 11 und 14 stellt die Distanz zwischen den beiden Elektronenaustrittsfenstern 5a und 5b dar und entspricht der Flächenmasse von etwa 230 g/m$^2$, was multipliziert mit der Dichte von Luft (hier angenommen 1188 g/m$^3$) etwa 196 mm entspricht. Erfindungsgemäß ergibt sich unter den angenommenen Bedingungen also ein optimaler Abstand von 196 mm, bei dem keine Leistung im jeweils gegenüberliegenden Elektronenaustrittsfenster absorbiert wird. Die Distanz kann entsprechend des Abstandsfaktors variiert werden.

Fig. 2 zeigt auch den Punkt 16 mit der höchsten Energiedosis, die bei etwa 100 g/m$^2$ durch den Elektronenbeschleuniger 3a erzeugt wird. In etwa an diesem Punkt sind die Elektronenreflektoren 7a1 und 7a2 angeordnet. Unter Berücksichtigung der als schraffierte Fläche 15 dargestellten Flächenmasse des Elektronenaustrittsfensters 5a von etwa 50 g/m$^2$ ergibt sich an Luft die optimale Distanz der Reflektoren 7a1 und 7a2 zum Elektronenaustrittsfenster 5a von etwa 42 mm. Gleiche Verhältnisse gelten für den Elektronenbeschleuniger 3b mit den Reflektoren 7b1 und 7b2.

Fig. 3 zeigt eine Detailansicht eines Reflektorsystems, umfassend die Reflektoren 7a1 und 7b1 aus Fig. 1, welche gleichzeitig als Bestandteile eines Sensorsystems ausgebildet sind. Aus Fig. 3 ist ersichtlich, das die Reflektoren 7a1 und 7b1 in y-Richtung, also in Bewegungsrichtung des Formteils 2, in Teilreflektoren 7a1.1 und 7a1.2 bzw. 7b1.1 und 7b1.2 unterteilt sind. Dabei ist jeder Teilreflektor gegenüber allen anderen Teilreflektoren elektrisch isoliert angeordnet. So wie dem Teilreflektor 7a1.1 eine Messeinrichtung 9a1.1 zugeordnet ist, ist auch jedem weiteren Teilreflektor eine Messeinrichtung zugeordnet, mittels der die auf dem zugeordneten Teilreflektor auftreffenden Elektronenströme erfassbar sind.

Wie eben in Bezug auf die Reflektoren 7a1 und 7b1 beschrieben, sind auch die zu den Reflektoren 7a1 und 7b1 spiegelsymmetrisch angeordneten Reflektoren 7a2 und 7b2 in Teilreflektoren unterteilt, welche gleichzeitig mit zugehörigen Messeinrichtungen Bestandteile eines Sensorsystems sind.

Auf diese Weise gibt es jeweils in x-, y- und z- Richtung mindestens zwei Messpunkte mit entsprechenden Messergebnissen, mittels denen somit eine Aussage bezüglich der Elektronenstromdichteverteilung in x-, y- und z-Richtung möglich ist. Dabei sollte erkennbar sein, dass eine umso genauere Aussage bezüglich der Elektronenstromdichteverteilung getroffen werden kann, je höher die Anzahl der in x-, y- und/oder z-Richtung ausgebildeten Teilreflektoren ist.

In Abhängigkeit von den auf diese Weise ermittelten Elektronenstromdichteverteilungen ist Vorrichtung 1 daher für die kontinuierliche Prozesskontrolle durch Überwachung und ggf. Steuerung der Strahlstromdichteverteilung der beiden gegenüberliegenden Elektronenbeschleuniger 3a und 3b geeignet. Mittels der erfindungsgemäßen Vorrichtung 1 ist es daher möglich, zum einen die gesamte Oberfläche eines Formteils 2 trotz nur zweier Elektronenaustrittsfenster 5a, 5b flächendeckend mit Elektronen zu beaufschlagen, andererseits ist dabei der Vorgang derart steuerbar, dass alle Oberflächenabschnitte mit einer weitgehend gleichmäßigen Energiedosis beaufschlagt werden.

Durch die Kombination aus Reflektor- und Sensorsystem ist es außerdem möglich, den Aufenthalt der Formteile 2 in der Prozesszone räumlich und zeitlich zu überwachen. Bei Abwesenheit eines Formteils 2 treffen die Randstrahlen 8 auf den jeweils gegenüberliegenden Reflektor (z. B. Randstrahl 8a1 auf Reflektor 7a1 und dann auf Reflektor 7a2) und werden im Sensorsystem als ansteigender Elektronenstromwert registriert. Bei Anwesenheit eines Formteils 2 in der Prozesszone absorbiert das Formteil 2 hingegen die reflektierten Randstrahlen und das registrierte Signal verringert sich. Zusätzlich verringert sich der Anteil an sonstigen Streuelektronen, die auf das Sensorsystem treffen. So kann eine Aussage getroffen werden, ob sich ein Formteil 2 im Prozessraum befindet.

## Patentansprüche

1. Vorrichtung zur Eigenschaftsänderung eines dreidimensionalen Formteils (2) mittels Elektronen, umfassend mindestens einen Elektronenbeschleuniger (3a; 3b) zum Erzeugen von beschleunigten Elektronen und zwei Elektronenaustrittsfenster (5a; 5b), wobei die beiden Elektronenaustrittsfenster (5a; 5b) gegenüberliegend angeordnet sind, **dadurch gekennzeichnet, dass** die beiden Elektronenaustrittsfenster (5a; 5b) und mindestens ein Reflektor (7a1; 7a2; 7b1; 7b2) einen Prozessraum begrenzen, in welchem die Oberfläche oder eine Randschicht des Formteils (2) mit Elektronen beaufschlagbar ist, wobei mittels eines Sensorsystems eine Energiedichteverteilung innerhalb des Prozessraumes zumindest über eine räumliche Dimension erfassbar ist.

2. Vorrichtung nach Anspruch 1, **dadurch gekennzeichnet, dass** die Flächen der Elektronenaustrittsfenster (5a; 5b) eben ausgebildet sind.

3. Vorrichtung nach Anspruch 1 oder 2, **dadurch gekennzeichnet, dass** die Flächen der Elektronenaustrittsfenster (5a; 5b) parallel zueinander ausgerichtet sind.

4. Vorrichtung nach Anspruch 1 oder 2, **dadurch gekennzeichnet, dass** die Flächen der Elektronenaustrittsfenster einen Winkel zueinander aufweisen.

5. Vorrichtung nach Anspruch 1, **dadurch gekennzeichnet, dass** die Fläche mindestens eines Elektronenaustrittsfensters zum Formteil hin konkav ausgebildet ist.

6. Vorrichtung nach Anspruch 1, **dadurch gekennzeichnet, dass** die Fläche mindestens eines Elektronenaustrittsfensters der Geometrie des Formteils angepasst ist.

7. Vorrichtung nach einem der vorhergehenden Ansprüche, **gekennzeichnet durch** eine erste Einrichtung, mittels der die über die Fläche mindestens eines Elektronenaustrittsfensters (5a; 5b) abgegebene Elektronenenergiedichte derart steuerbar ist, dass über einzelne Teilbereiche des Elektronenaustrittsfensters (5a, 5b) unterschiedliche Elektronenenergiedichten abgegeben werden.

8. Vorrichtung nach einem der vorhergehenden Ansprüche, **dadurch gekennzeichnet**, das mindestens zwei Reflektoren (7a1 zu 7a2 und 7b1 zu 7b2) spiegelsymmetrisch an gegenüberliegenden Seiten des Prozessraumes angeordnet sind.

**9.** Vorrichtung nach Anspruch 8, **dadurch gekennzeichnet, dass** die Reflektoren (7a1; 7a2; 7b1; 7b2) Bestandteile eines Sensorsystems zum Erfassen einer Energiedichteverteilung innerhalb des Prozessraumes sind.

**10.** Vorrichtung nach Anspruch 9, **dadurch gekennzeichnet, dass** an mindestens zwei Reflektoren oder Teilreflektoren die elektrische Spannung gegenüber der elektrischen Masse oder einem anderen elektrischen Potential erfassbar ist.

**11.** Vorrichtung nach einem der Ansprüche 8 bis 10, **dadurch gekennzeichnet, dass** die Energiedichteverteilung in x-, y- und/oder z-Richtung eines rechtwinkligen Koordinatensystems erfassbar ist

**12.** Vorrichtung nach einem der vorhergehenden Ansprüche, **dadurch gekennzeichnet, dass** mindestens ein Elektronenaustrittsfenster in Abhängigkeit von der Geometrie des Formteils oder/und der Position des Formteils zwischen den Elektronenaustrittsfenstern bewegbar ist.

**13.** Vorrichtung nach einem der vorhergehenden Ansprüche, **dadurch gekennzeichnet, dass** mindestens ein Elektronenaustrittsfenster (5a; 5b) als vakuumdichte Folie ausgebildet ist.

**14.** Vorrichtung nach einem der vorhergehenden Ansprüche, **dadurch gekennzeichnet, dass** mindestens ein Elektronenaustrittsfenster als gasdurchlässige Druckstufenanordnung zwischen Elektronenbeschleuniger und Prozessraum ausgebildet ist.

**15.** Vorrichtung nach einem der vorhergehenden Ansprüche, **gekennzeichnet durch** ein Sensorsystem, mittels dem die Leistung der Elektronenerzeuger in Abhängigkeit davon, ob sich ein Formteil im Prozessraum befindet, auf einen prozessspezifischen Wert einstellbar ist.

**16.** Vorrichtung nach einem der vorhergehenden Ansprüche, **gekennzeichnet durch** eine zweite Einrichtung, mittels der die Austrittsrichtung eines Elektrons aus einem Elektronenaustrittsfenster steuerbar ist.

**17.** Vorrichtung nach einem der vorhergehenden Ansprüche, **dadurch gekennzeichnet, dass** der Elektronenbeschleuniger als Bandstrahler oder Axialstrahlerzeuger ausgebildet ist.

**18.** Vorrichtung nach einem der vorhergehenden Ansprüche, **dadurch gekennzeichnet, dass** die Elektronenaustrittsfenster (5a; 5b) einen Abstand a aufweisen, der sich in einem Bereich befindet resultierend aus der Berechnung

$$a = f * \frac{6{,}67 * 10^{-7} \frac{(Ub * k_1)^{5/3}}{\rho_W} * k_2 - \rho_F * d_F}{\rho_G}$$

Ub = Beschleunigungsspannung
$\rho_W$ = Dichte von Wasser
$\rho_G$ = Dichte des Mediums zwischen den Elektronenaustrittsfenstern $\rho_F$ = Dichte der Fensterfolie
$d_F$ = Dicke der Fensterfolie
$k_1 = 1 * V^{-1}$
$k_2 = 1 * (g/m^2)^{-1}$
mit einem Abstandsfaktor f (0,5 < f < 1, 5).

**19.** Verfahren zur Eigenschaftsänderung eines dreidimensionalen Formteils (2) mittels Elektronen, bei dem mittels mindestens einem Elektronenbeschleuniger (3a; 3b) Elektronen erzeugt, beschleunigt und über die Fläche von zwei gegenüberliegenden Elektronenaustrittsfenstern (5a; 5b) abgestrahlt werden, **dadurch gekennzeichnet, dass** die beiden Elektronenaustrittsfenster (5a; 5b) und mindestens ein Reflektor (7a1; 7a2; 7b1; 7b2) einen Prozessraum begrenzen, in welchem die Oberfläche oder eine Randschicht des Formteils (2) mit Elektronen beaufschlagt wird, wobei mittels eines Sensorsystems eine Energiedichteverteilung innerhalb des Prozessraumes zumindest über eine räumliche Dimension erfasst und der Abstand der Elektronenaustrittsfenster derart eingestellt wird, dass eine Beeinflussung eines Elektronenaustrittsfensters (5a; 5b) durch die abgestrahlte Energie des gegenüberliegenden Elektronenaustrittsfensters (5b; 5a) vernachlässigbar ist.

**20.** Verfahren nach Anspruch 19, **dadurch gekennzeichnet, dass** der Abstand der Elektronenaustrittsfenster in Abhängigkeit von der Beschleunigungsspannung der Elektronen und der Dicke und der Dichte der Elektronenaustrittsfenster (5a; 5b) eingestellt wird.

**21.** Verfahren nach Anspruch 20, **dadurch gekennzeichnet, dass** der Abstand a der Elektronenaustrittsfenster (5a; 5b) in einem Bereich eingestellt wird, der sich aus

$$a = f * \frac{6{,}67 * 10^{-7} \frac{(Ub * k_1)^{5/3}}{\rho_W} * k_2 - \rho_F * d_F}{\rho_G}$$

Ub = Beschleunigungsspannung
$\rho_W$ = Dichte von Wasser
$\rho_G$ = Dichte des Mediums zwischen den Elektronenaustrittsfenstern $\rho_F$ = Dichte der Fensterfolie
$d_F$ = Dicke der Fensterfolie
$k_1 = 1*V^{-1}$
$k_2 = 1*(g/m^2)^{-1}$
mit einem Abstandsfaktor f ($0{,}5 < f < 1{,}5$) ergibt.

**22.** Verfahren nach Anspruch 19 oder 21, **dadurch gekennzeichnet, dass** das Formteil (2) mit konstanter Geschwindigkeit durch den Prozessraum geführt und währenddessen mit Elektronen beaufschlagt wird.

**23.** Verfahren nach Anspruch 19 oder 21, **dadurch gekennzeichnet, dass** das Formteil in den Prozessraum geführt und dort im stationären Zustand durch einen einmaligen oder mehrfachen Bestrahlungsvorgang mit Elektronen beaufschlagt wird.

**24.** Verfahren nach Anspruch 19 oder 21, **dadurch gekennzeichnet, dass** das Formteil im Step-and-Repeat-Modus mit Elektronen beaufschlagt wird.

**25.** Verfahren nach Anspruch 19 oder 21, **dadurch gekennzeichnet, dass** das Formteil im Prozessraum um eine sich zwischen den beiden Elektronenaustrittsfenstern erstreckende Achse rotiert und währenddessen durch einen einmaligen oder mehrfachen Bestrahlungsvorgang mit Elektronen beaufschlagt wird.

**26.** Verwendung eines Verfahrens nach einem der vorhergehenden Ansprüche zum Modifizieren von Kunststoffen, Sterilisieren von Produkten/Zwischenprodukten der Pharmaindustrie, Desinfizieren oder/und Sterilisieren von Verpackungen, Härten von Beschichtungen oder Desinfizieren oder/und Sterilisieren von Gegenständen, Früchten oder anderen Naturprodukten.

## Claims

**1.** Apparatus for changing the properties of a three-dimensional shaped part (2) by means of electrons, comprising at least one electron accelerator (3a; 3b) for generating accelerated electrons and two electron exit windows (5a; 5b), wherein the two electron exit windows (5a; 5b) are arranged opposite one another, **characterized in that** the two electron exit windows (5a; 5b) and at least one reflector (7a1; 7a2; 7b1; 7b2) delimit a process space in which the surface or a peripheral layer of the shaped part (2) can be subjected to electrons, wherein an energy density distribution within the process space at least over one spatial dimension can be detected by means of a sensor system.

**2.** Apparatus according to Claim 1, **characterized in that** the surfaces of the electron exit windows (5a; 5b) have a planar configuration.

**3.** Apparatus according to Claim 1 or 2, **characterized in that** the surfaces of the electron exit windows (5a; 5b) have a mutually parallel orientation.

**4.** Apparatus according to Claim 1 or 2, **characterized in that** the surfaces of the electron exit windows are at an angle with respect to each other.

5. Apparatus according to Claim 1, **characterized in that** the surface of at least one electron exit window is configured to be concave in the direction of the shaped part.

6. Apparatus according to Claim 1, **characterized in that** the surface of at least one electron exit window is matched to the geometry of the shaped part.

7. Apparatus according to one of the preceding claims, **characterized by** a first device, by means of which the electron energy density emitted via the surface of at least one electron exit window (5a; 5b) can be controlled such that different electron energy densities are emitted via individual partial regions of the electron exit window (5a; 5b).

8. Apparatus according to one of the preceding claims, **characterized in that** at least two reflectors (7a1 to 7a2 and 7b1 to 7b2) are arranged in mirror-symmetric fashion on opposite sides of the process space.

9. Apparatus according to Claim 8, **characterized in that** the reflectors (7a1; 7a2; 7b1; 7b2) are components of a sensor system for detecting an energy density distribution within the process space.

10. Apparatus according to Claim 9, **characterized in that** at at least two reflectors or partial reflectors the electric voltage with respect to electrical ground or another electric potential can be detected.

11. Apparatus according to one of Claims 8 to 10, **characterized in that** the energy density distribution can be detected in the x, y and/or z direction of a rectangular coordinate system.

12. Apparatus according to one of the preceding claims, **characterized in that** at least one electron exit window is moveable depending on the geometry of the shaped part and/or on the position of the shaped part between the electron exit windows.

13. Apparatus according to one of the preceding claims, **characterized in that** at least one electron exit window (5a; 5b) is configured as a vacuum-tight film.

14. Apparatus according to one of the preceding claims, **characterized in that** at least one electron exit window is configured as a gas-permeable pressure-level arrangement between electron accelerator and process space.

15. Apparatus according to one of the preceding claims, **characterized by** a sensor system, by means of which the output of the electron generators can be adjusted to a process-specific value depending on whether a shaped part is located in the process space.

16. Apparatus according to one of the preceding claims, **characterized by** a second device, by means of which the exit direction of an electron from an electron exit window can be controlled.

17. Apparatus according to one of the preceding claims, **characterized in that** the electron accelerator is configured as a band emitter or axial beam generator.

18. Apparatus according to one of the preceding claims, **characterized in that** the electron exit windows (5a; 5b) have a spacing a, which is located in a region resulting from the following calculation

$$a = f * \frac{6.67 * 10^{-7} \frac{(Ub * k_1)^{5/3}}{\rho_W} * k_2 - \rho_F * d_F}{\rho_G}$$

Ub = acceleration voltage
$\rho_W$ $\rho_W$ = density of water
$\rho_G$ $P_G$ = density of the medium between the electron exit windows
$\rho_F$ $\rho_F$ = density of the window film

$d_F$ = thickness of the window film
$k_1 = 1*V^{-1}$
$k_2 = 1*(g/m^2)^{-1}$
with a spacing factor f (0.5 < f < 1.5).

**19.** Method for changing the properties of a three-dimensional shaped part (2) by means of electrons, in which at least one electron accelerator (3a; 3b) is used to generate electrons, accelerate them and emit them via the surface of two opposite electron exit windows (5a; 5b), **characterized in that** the two electron exit windows (5a; 5b) and at least one reflector (7a1; 7a2; 7b1; 7b2) delimit a process space in which the surface or a peripheral layer of the shaped part (2) is subjected to electrons, wherein an energy density distribution within the process space at least over one spatial dimension is detected by means of a sensor system and the spacing of the electron exit windows is adjusted such that any influence on an electron exit window (5a; 5b) by the emitted energy of the opposite electron exit window (5b; 5a) is negligible.

**20.** Method according to Claim 19, **characterized in that** the spacing between the electron exit windows is adjusted as a function of the acceleration voltage of the electrons and the thickness and the density of the electron exit windows (5a; 5b).

**21.** Method according to Claim 20, **characterized in that** the spacing a between the electron exit windows (5a; 5b) is adjusted in a region resulting from

$$a = f * \frac{6.67 * 10^{-7} \dfrac{(Ub * k_1)^{5/3}}{\rho_W} * k_2 - \rho_F * d_F}{\rho_G}$$

Ub = acceleration voltage
$\rho_W$ $\rho_W$ = density of water
$\rho_G$ $P_G$ = density of the medium between the electron exit windows
$\rho_F$ $\rho_F$ = density of the window film
$d_F$ = thickness of the window film
$k_1 = 1*V^{-1}$
$k_2 = 1*(g/m^2)^{-1}$
with a spacing factor f (0.5 < f < 1.5).

**22.** Method according to Claim 19 or 21, **characterized in that** the shaped part (2) is guided through the process space at a constant speed and while doing so is subjected to electrons.

**23.** Method according to Claim 19 or 21, **characterized in that** the shaped part is guided into the process space and is subjected there in the stationary state to electrons by a single irradiation operation or by multiple irradiation operations.

**24.** Method according to Claim 19 or 21, **characterized in that** the shaped part is subjected to electrons in a step-and-repeat mode.

**25.** Method according to Claim 19 or 21, **characterized in that** the shaped part in the process space rotates about an axis extending between the two electron exit windows and while doing so is subjected to electrons by a single irradiation operation or by multiple irradiation operations.

**26.** Use of a method according to one of the preceding claims for modifying plastics, sterilizing products/intermediates of the pharmaceutical industry, disinfecting or/and sterilizing packages, curing coatings or disinfecting or/and sterilizing objects, fruit or other natural products.

## Revendications

1. Dispositif pour modifier les propriétés d'un corps formé (2) tridimensionnel au moyen d'électrons, comprenant au moins un accélérateur d'électrons (3a ; 3b) pour générer des électrons accélérés et deux fenêtres de sortie d'électrons (5a; 5b), les deux fenêtres de sortie d'électrons (5a; 5b) étant disposées l'une en face de l'autre, **caractérisé en ce que** les deux fenêtres de sortie d'électrons (5a; 5b) et au moins un réflecteur (7a1; 7a2 ; 7be1; 7b2) délimitent une chambre de processus dans laquelle la surface ou une couche marginale du corps formé (2) peut être exposée à des électrons, une répartition de densité d'énergie à l'intérieur de la chambre de processus au moins dans une dimension spatiale pouvant être enregistrée au moyen d'un système capteur.

2. Dispositif selon la revendication 1, **caractérisé en ce que** les surfaces des fenêtres de sortie d'électrons (5a; 5b) sont conçues planes.

3. Dispositif selon la revendication 1 ou 2, **caractérisé en ce que** les surfaces des fenêtres de sortie d'électrons (5a; 5b) sont orientées parallèlement entre elles.

4. Dispositif selon la revendication 1 ou 2, **caractérisé en ce que** les surfaces des fenêtres de sortie d'électrons présentent un certain angle les unes par rapport aux autres.

5. Dispositif selon la revendication 1, **caractérisé en ce que** la surface d'au moins une fenêtre de sortie d'électrons est conçue concave en direction du corps formé.

6. Dispositif selon la revendication 1, **caractérisé en ce que** la surface d'au moins une fenêtre de sortie d'électrons est adaptée à la géométrie du corps formé.

7. Dispositif selon l'une quelconque des revendications précédentes, **caractérisé par** un premier dispositif, au moyen duquel la densité d'énergie d'électrons délivrée par la surface d'au moins une fenêtre de sortie d'électrons (5a ; 5b) peut être contrôlée de telle sorte que différentes densités d'énergie d'électrons soient délivrées par des zones partielles individuelles de la fenêtre de sortie d'électrons (5a; 5b).

8. Dispositif selon l'une quelconque des revendications précédentes, **caractérisé en ce qu'**au moins deux réflecteurs (7a1/7a2 et 7b1/7b2) sont disposés de façon symétrique sur des côtés opposés de la chambre de processus.

9. Dispositif selon la revendication 8, **caractérisé en ce que** les réflecteurs (7a1; 7a2; 7b1; 7b2) sont des composants d'un système capteur pour l'enregistrement d'une répartition de densité d'énergie à l'intérieur de la chambre de processus.

10. Dispositif selon la revendication 9, **caractérisé en ce que** la tension électrique par rapport à la masse électrique ou à un autre potentiel électrique peut être enregistrée sur au moins deux réflecteurs ou réflecteurs partiels.

11. Dispositif selon l'une quelconque des revendications 8 à 10, **caractérisé en ce que** la répartition de densité d'énergie dans la direction x, y et/ou z d'un système de coordonnées orthogonal peut être enregistrée.

12. Dispositif selon l'une quelconque des revendications précédentes, **caractérisé en ce qu'**au moins une fenêtre de sortie d'électrons peut être déplacée en fonction de la géométrie du corps formé et/ou de la position du corps formé entre les fenêtres de sortie d'électrons.

13. Dispositif selon l'une quelconque des revendications précédentes, **caractérisé en ce qu'**au moins une fenêtre de sortie d'électrons (5a ; 5b) est conçue sous forme de film étanche au vide.

14. Dispositif selon l'une quelconque des revendications précédentes, **caractérisé en ce qu'**au moins une fenêtre de sortie d'électrons est conçue sous forme d'agencement à niveaux de pression perméable au gaz entre l'accélérateur d'électrons et la chambre de processus.

15. Dispositif selon l'une quelconque des revendications précédentes, **caractérisé par** un système capteur, au moyen duquel la puissance des générateurs d'électrons peut être réglée sur une valeur spécifique au processus selon qu'un corps formé se trouve ou non dans la chambre de processus.

**16.** Dispositif selon l'une quelconque des revendications précédentes, **caractérisé par** un second dispositif, au moyen duquel le sens de sortie d'un électron d'une fenêtre de sortie d'électrons peut être commandé.

**17.** Dispositif selon l'une quelconque des revendications précédentes, **caractérisé en ce que** l'accélérateur d'électrons est conçu sous forme d'élément rayonnant à bande ou de générateur de faisceau axial.

**18.** Dispositif selon l'une quelconque des revendications précédentes, **caractérisé en ce que** les fenêtres de sortie d'électrons (5a; 5b) présentent un espacement a qui se situe dans une plage résultant de l'équation suivante

$$a = f * \frac{6{,}67 * 10^{-7} \frac{(Ub * k_1)^{5/3}}{\rho_W} * k_2 - \rho_F * d_F}{\rho_G}$$

Ub = tension d'accélération
$\rho_W$ = densité de l'eau
$\rho_G$ = densité du fluide entre les fenêtres de sortie d'électrons
$\rho_F$ = densité du film de fenêtre
$d_F$ = épaisseur du film de fenêtre
$k_1 = 1*V^{-1}$
$k_2 = 1*(g/m^2)^{-1}$
avec un facteur d'espacement f ($0{,}5 < f < 1{,}5$).

**19.** Procédé pour modifier les propriétés d'un corps formé (2) tridimensionnel au moyen d'électrons, dans lequel des électrons sont générés au moyen d'au moins un accélérateur d'électrons (3a; 3b), accélérés et émis par la surface de deux fenêtres de sortie d'électrons (5a; 5b) opposées, **caractérisé en ce que** les deux fenêtres de sortie d'électrons (5a; 5b) et au moins un réflecteur (7a1; 7a2; 7b1; 7b2) délimitent une chambre de processus dans laquelle la surface ou une couche marginale du corps formé (2) est exposée à des électrons, une répartition de densité d'énergie à l'intérieur de la chambre de processus au moins dans une dimension spatiale étant enregistrée au moyen d'un système capteur et l'espacement des fenêtres de sortie d'électrons étant réglé de telle sorte qu'une influence d'une fenêtre de sortie d'électrons (5a; 5b) par l'énergie émise de la fenêtre de sortie d'électrons (5a; 5b) opposée peut être négligée.

**20.** Procédé selon la revendication 19, **caractérisé en ce que** l'espacement des fenêtres de sortie d'électrons est réglé en fonction de la tension d'accélération des électrons et de l'épaisseur et de la densité des fenêtres de sortie d'électrons (5a; 5b).

**21.** Procédé selon la revendication 20, **caractérisé en ce que** l'espacement a des fenêtres de sortie d'électrons (5a; 5b) est réglé dans une plage qui est obtenue à partir de l'équation suivante

$$a = f * \frac{6{,}67 * 10^{-7} \frac{(Ub * k_1)^{5/3}}{\rho_W} * k_2 - \rho_F * d_F}{\rho_G}$$

Ub = tension d'accélération
$\rho_W$ = densité de l'eau
$\rho_G$ = densité du fluide entre les fenêtres de sortie d'électrons
$\rho_F$ = densité du film de fenêtre
$d_F$ = épaisseur du film de fenêtre
$k_1 = 1*V^{-1}$
$k_2 = 1*(g/m2)^{-1}$
avec un facteur d'espacement f ($0{,}5 < f < 1{,}5$).

**22.** Procédé selon la revendication 19 ou 21, **caractérisé en ce que** le corps formé (2) est guidé à vitesse constante à travers la chambre de processus et est exposé pendant ce temps à des électrons.

**23.** Procédé selon la revendication 19 ou 21, **caractérisé en ce que** le corps formé est guidé dans la chambre de processus et y est exposé à des électrons, dans l'état stationnaire, par une opération d'irradiation unique ou multiple.

**24.** Procédé selon la revendication 19 ou 21, **caractérisé en ce que** le corps formé est exposé à des électrons dans le mode "Step-and-Repeat".

**25.** Procédé selon la revendication 19 ou 21, **caractérisé en ce que** le corps formé tourne dans la chambre de processus autour d'un axe s'étendant entre les deux fenêtres de sortie d'électrons et est exposé à des électrons pendant ce temps par une opération d'irradiation unique ou multiple.

**26.** Utilisation d'un procédé selon l'une quelconque des revendications précédentes pour la modification de plastiques, la stérilisation de produits/produits intermédiaires de l'industrie pharmaceutique, la désinfection et/ou la stérilisation d'emballages, le durcissement de revêtements ou la désinfection et/ou la stérilisation d'objets, de fruits ou d'autres produits naturels.

Fig. 1

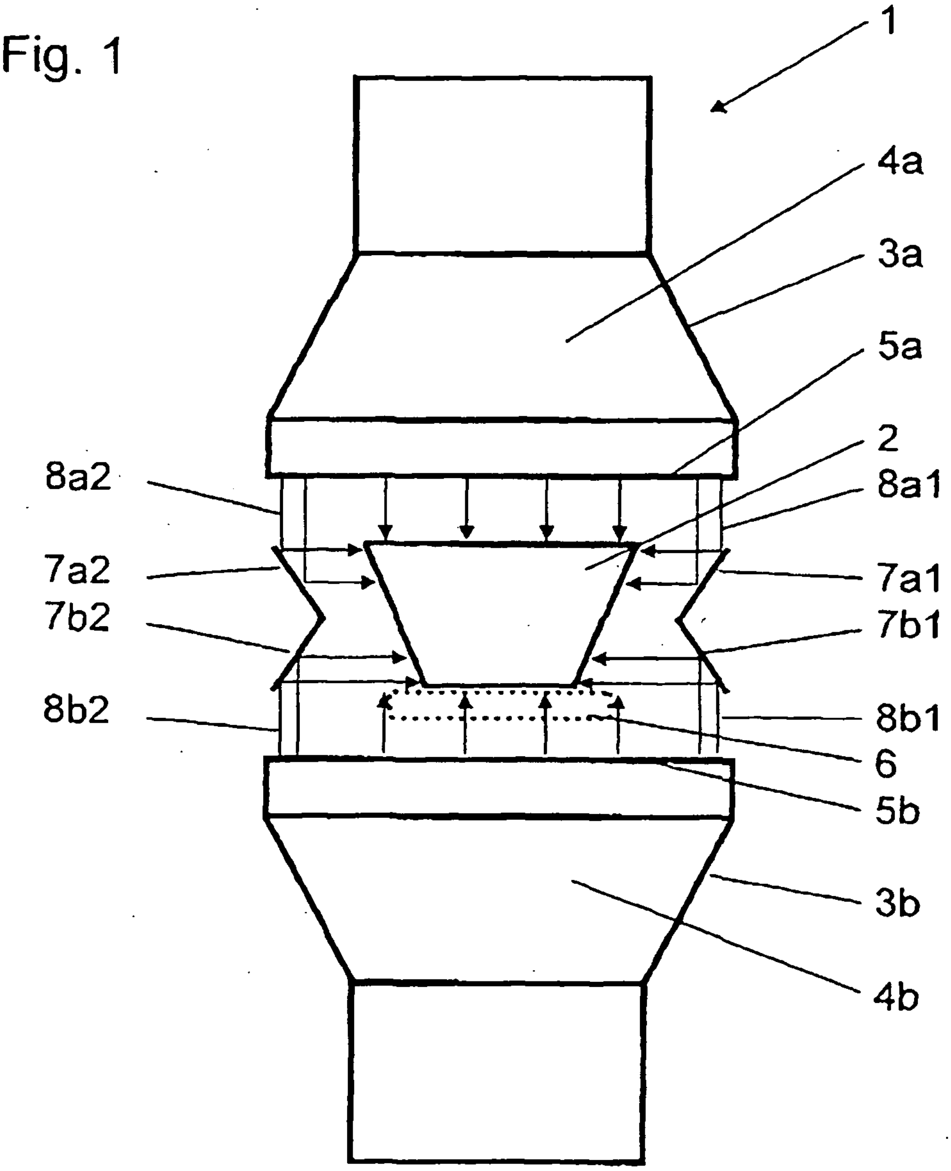

Fig. 2

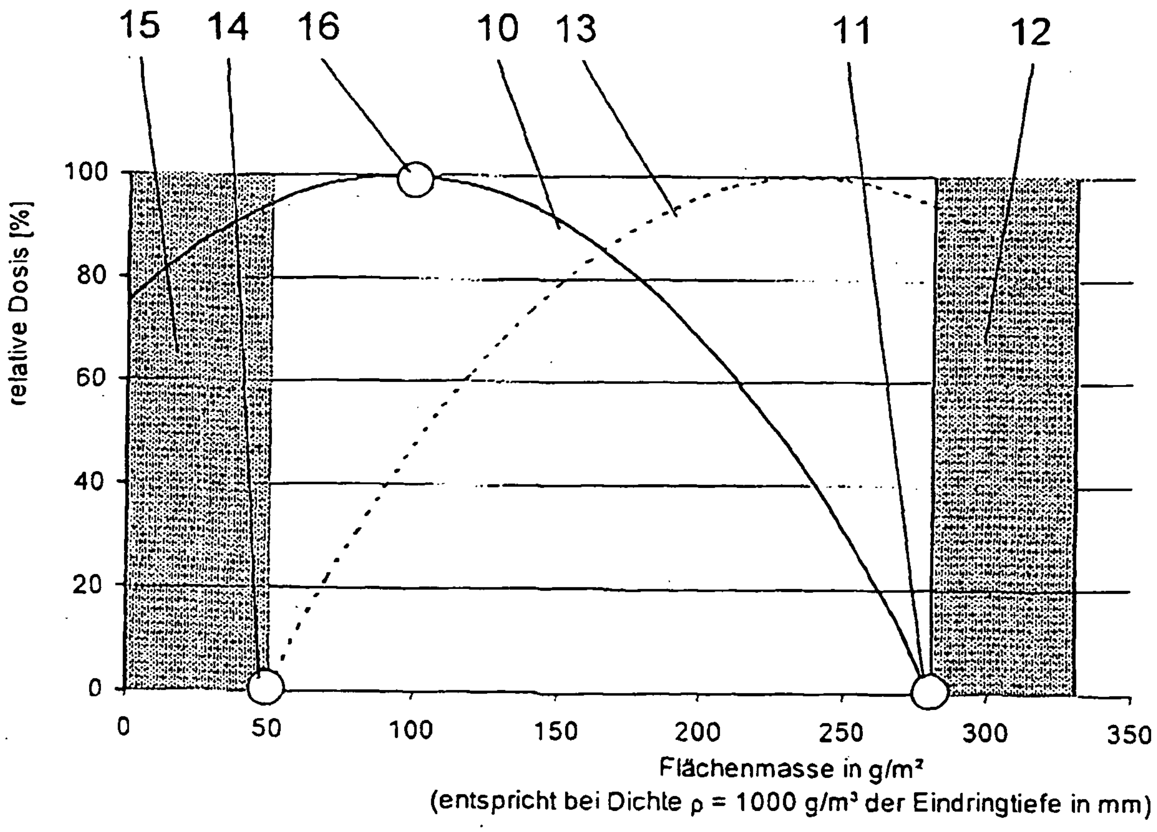
15
14
16
10
13
11
12
relative Dosis [%]
100
80
60
40
20
0
0
50
100
150
200
250
300
350
Flächenmasse in g/m²
(entspricht bei Dichte ρ = 1000 g/m³ der Eindringtiefe in mm)

Fig. 3

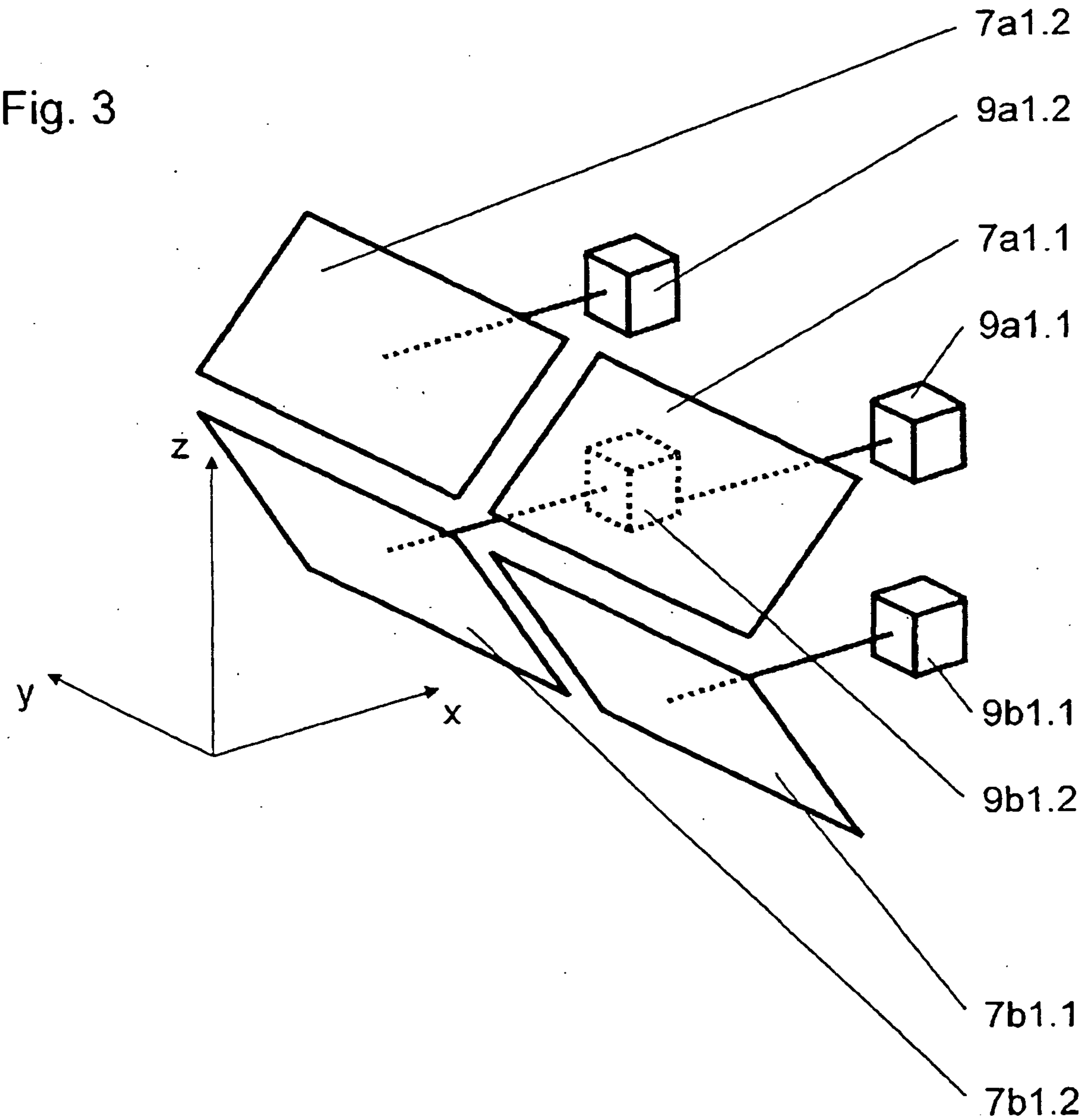

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19942142 A1
- US 2741704 A